# EUROPÄISCHE PATENTSCHRIFT

(11) **EP 1 017 741 B1**
(45) Veröffentlichungstag und Bekanntmachung des Hinweises auf die Patenterteilung: **30.05.2001**
(21) Anmeldenummer: 98945275.0
(22) Anmeldetag: 21.08.1998
(51) Int. Cl.: C08J 9/28, A61K 9/70

(54) **TREIBMITTELFREIES VERFAHREN ZUR HERSTELLUNG FLÄCHENFÖRMIGER POLYMERSCHÄUME**
NON-EXPANDING AGENT METHOD FOR PRODUCING FLAT-SHAPED POLYMER FOAMS
PROCEDE DE PRODUCTION DE MOUSSES POLYMERES PLANIFORMES, NE FAISANT PAS APPEL A UN AGENT POROGENE

(30) Priorität: 05.09.1997 DE 19738856
(43) Veröffentlichungstag der Anmeldung: 12.07.2000
(73) Patentinhaber: LTS Lohmann Therapie-Systeme AG, 56626 Andernach (DE)
(72) Erfinder: BRACHT, Stefan, D-56299 Ochtendung (DE)
(74) Vertreter: Flaccus, Rolf-Dieter, Dr.
(86) Internationale Anmeldenummer: EP9805322
(87) Internationale Veröffentlichungsnummer: WO9913001

(56) Entgegenhaltungen:
- EP-A- 0 334 998
- US-A- 4 820 525

## Beschreibung

Schäumungsverfahren finden insbesondere in der Kunststoffverarbeitung sehr breite Anwendung. Schäume werden hauptsächlich zu einer Reduktion des Materialverbrauchs, zu einer Gewichtsreduktion des Produktes oder zur Beeinflussung innerer Materialeigenschaften wie insbesondere Steifigkeit, Elastizität, Sprödigkeit, Wärmeleitfähigkeit und Schallisolierung eingesetzt. Im Bereich medizinischer Produkte zur äußerlichen Anwendung werden Schäume wegen ihrer verbesserten Elastizität und Flexibilität eingesetzt. Offene Schäume, also zweiphasige Systeme mit einem bikohärenten Aufbau, eignen sich als Trägerstoff zur Einbettung einer flüssigen, halbfesten oder auch festen Matrix. Offene Schäume als Trägerstoff finden auch Anwendung bei innerlich anzuwendenden medizinischen Produkten, wie z.B. Tabletten oder Implantaten.

Gegenstand der vorliegenden Erfindung ist ein Verfahren zur Herstellung vorzugsweise geschlossener Schäume sowie diese Schäume enthaltende transdermale therapeutische Systeme (TTS).

Zur Herstellung von Schäumen werden in der Kunststoffindustrie hauptsächlich feste und flüssige Treibmittel eingesetzt. Daneben besitzt das Phaseninversionsverfahren Bedeutung. Einen wichtigen Sonderfall stellt die gleichzeitige Nutzung von Reaktionsgasen der Polymerisation zur Schäumung von Polyurethanen dar. Feste chemische Treibmittel sind Stoffe, die durch thermische Zersetzung ab einer bestimmten Temperatur mehr oder weniger vollständig in gasförmige Produkte zerfallen. Das in einer Polymermasse dispers eingebettete Treibmittel führt auf diesem Wege zur Schäumung des Materials. Ein solches Verfahren erfordert relativ hohe Verarbeitungstemperaturen von nach jetzigem Stand der Technik mindestens 100°C, typischerweise sogar oberhalb 140°C. Darüberhinaus hinterlassen die meisten dieser Treibmittel Rückstände im Produkt.

Insbesondere im medizinischen Bereich stellen hohe Verarbeitungstemperaturen eine Gefährdung der Stabilität von pharmazeutischen Wirk- und Hilfsstoffen dar. Die Treibmittelrückstände sind toxikologisch schwer zu beurteilen und stellen im international unterschiedlichen Zulassungswesen ein Hemmnis und Risiko dar.

Als flüssige Treibmittel lassen sich unter Druck verflüssigte Gase einsetzen. Die im Produkt dispergierte Flüssigkeit wird anschließend unter Normaldruck gebracht, geht in den Gaszustand über und führt so zur Schaumbildung. Klassische Einsatzstoffe sind auf diesem Gebiet die Fluorchlorkohlenwasserstoffe(FCKW). Sie werden wegen der bekannten Umweltproblematik zunehmend durch fluorierte Kohlenwasserstoffe(FKW), halogenfreie Kohlenwasserstoffe (KW; Alkane) und aktuell auch durch Kohlendioxyd ersetzt.

Schäumungsverfahren unter Verwendung flüssiger Treibmittel sind technisch vergleichsweise aufwendig, da die Herstellung und Verarbeitung der Masse in Teilen unter erheblichem Überdruck stattfinden muß.
Ein Sonderfall der treibmittelbasierten Schäumungsverfahren findet sich bei den Polyurethanen. Die bei der Polymerisation eingesetzten Isocyanate spalten in Anwesenheit von Wasser Kohlendioxyd ab, das gleichzeitig als Treibgas dient. Die Anwendung ist jedoch auf diese Stoffklasse beschränkt.

Neben diesen Standardverfahren existieren treibmittelfreie Verfahren zur Herstellung von Polymerschäumen. So kann aus einer losen Ansammlung von Kunststoffpartikeln (die für sich bereits geschäumt sein können) durch Hitzeeinwirkung ein poröses, schaumförmiges Aggregat erschmolzen werden. Technisch gelingt dies z.B. mit überhitztem Wasserdampf durch einen Autoklavierprozeß. Nachteilig ist neben der hohen Temperaturbelastung vor allem die Beschränkung auf Polymerwerkstoffe, die pulverförmig handhabbar sind. Viele Elastomere zeigen jedoch starke Adhäsion, klebrige Polymere können überhaupt nicht verarbeitet werden.

Beim Phaseninversionsverfahren kommt eine Emulsion zum Einsatz. Die innere, disperse Phase besteht aus dem zu verarbeitenden Polymer oder seiner Mischung mit anderen Stoffen. Die äußere Phase wird während des Prozesses durch Trocknung entfernt, wobei die innere Phase zuletzt schaumförmige Aggregate bildet. Die disperse innere Phase ist damit zu einer kohärenten Phase invertiert worden. Die entstehenden Schäume sind vorzugsweise offenporig und besitzen daher hohe Durchlässigkeit für Gase und Wasserdampf. Weiterhin ist der Volumenanteil der Hohlräume relativ gering, da der Hauptanteil des Schaumes aus zusammengedrängten Polymerpartikeln besteht.

Der Erfindung liegt die Aufgabe zugrunde, ein Verfahren zur Herstellung geschlossen-zelliger, schichtförmiger Schäume für die medizinische Anwendung bereitzustellen. Hohe Verarbeitungstemperaturen und der Einsatz gesundheitlich oder umwelttechnisch bedenklicher Treibmittel sollen vermieden werden. Das Verfahren soll sich weiterhin auf die Herstelltechnik des lösemittelhaltigen Beschichtens auf einen bahnförmigen Träger und der anschließenden Trocknung anwenden lassen.

Eine weitere Aufgabe der Erfindung besteht darin, schichtförmige Polymerprodukte mit einer rauhen Oberfläche herzustellen. Diese Produkte zeigen insbesondere bei der Kaschierung mit schichtförmigen, elastischen oder selbstklebenden Fremdpolymeren einen verbesserten mechanischen Verbund.
Diese Aufgaben werden gelöst durch ein treibmittelfreies Verfahren auf der Basis einer Emulsion, eines dispersen Systems vom Typ Flüssig/Flüssig gemäß den Merkmalen des Hauptanspruchs. Die äußere, kohärente Phase enthält das Polymer, während die innere disperse Phase bei der Verarbeitung durch Trocknung entfernt wird. Der Trocknungsvorgang kann zwei Phasen umfassen: Im ersten Teil der Trocknung wird die äußere polymerhaltige Phase vom flüssigen in einen hochviskosen, halbfesten oder festen Zustand überführt, je nach Eigenschaften des trockenen Polymers. Die disperse innere Phase verändert ihre Zusammensetzung dabei gar nicht oder nur in geringem Ausmaß. Lediglich ihr Volumenanteil nimmt durch den Volumenverlust der äußeren Phase zwangsläufig zu. In der zweiten Phase der Trocknung wird, nachdem sich die äußere Phase mechanisch verfestigt hat, die innere Phase durch Trocknung entfernt. Die Verfestigung der äußeren Phase kann durch Verflüchtigung von Lösemittel allein erfolgen. Zusätzlich können Quervernetzungsmittel eingesetzt werden, die entweder bei der Trocknung thermisch aktiviert werden oder aber durch Einstrahlung entsprechend energiereicher Lichtquanten.
Die Bestandteile der inneren Phase entweichen maßgeblich durch Diffusion in der äußeren Phase und Verdampfung an der Oberfläche. Dieser Prozeß ist bei geringen Schichtdicken leicht möglich, so daß das Verfahren sich speziell für schichtförmige Produkte eignet.

Dieses Verfahren vereint gegenüber den bekannten Verfahren eine Anzahl von Vorteilen. Dies gilt insbesondere für Schäumungsverfahren im Bereich medizinischer Produkte:
- Die hohen Temperaturen und die gesundheitlichen Risiken bei Einsatz fester chemischer Treibmittel werden vermieden.
- Es muß nicht unter Überdruck gearbeitet werden; umweltkritische flüssige Treibmittel sind nicht erforderlich.
- Es können auch Polymerschäume aus stark elastischen oder klebrigen Rohstoffen hergestellt werden, die sich bei Raumdruck und Raumtemperatur nicht pulverförmig verarbeiten lassen.
- Die Schäume können hohe Anteile an Hohlräumen enthalten.
- Die Polymerphase bildet eine kohärente und völlig homogene Schicht, frei von durch Verwachsung ursprünglich disperser Anteile entstandenen Nahtstellen oder Poren.

Darüberhinaus ergeben sich Spezialanwendungen, die nur mit diesem Verfahren möglich sind und im folgenden im einzelnen beschrieben werden.

Bei den zu schäumenden Polymeren handelt es sich vorzugsweise um Elastomere. Weiterhin bevorzugt handelt es sich um wasserunlösliche Polymere, die jedoch in organischen Lösungsmitteln gelöst werden können.

### Solche Polymere sind insbesondere:

Polyurethane, Ethylvinylacetat-Copolymere, Acrylatvinylpyrrolidon-Copolymere, Polyacrylate, Polymethacrylate, Polystyrol-basierte Kunstkautschuke (insbesondere Styrol-Isobutylen-Styrol-Blockcopolymere SIS und Styrol-Butadien-Styrol-Blockcopolymere SBS), reine Kohlenwasserstoffe wie Polyisobutylene, Polyisoprene sowie Silikon-kautschuke. Bei allen diesen Polymeren kann es sich auch um haftklebende oder heißschmelzklebende Modifizierungen oder Mischungen davon handeln.
Diese Polymere werden in Form hochviskoser Lösungen (dynamische Viskosität vorzugsweise 0,5 - 10 Pa·s) in organischen Lösungsmitteln verarbeitet. Organische Lösungsmittel für die äußere Phase sind vorzugsweise Isopropanol, Aceton, Methylethylketon, Ethylacetat, Diethylether, Diisopropylether, Cyclohexan, Hexan, Heptan, Octan, Petrolether und Toluol sowie Benzin verschiedener Siedebereiche.

In dieser Polymerlösung wird mit Hilfe geeigneter Dispergierwerkzeuge eine mit der Lösung praktisch nicht oder nur wenig mischbare Flüssigkeit eingebracht und tröpfchenförmig als innere Phase darin zerteilt.
Diese als innere Phase vorliegende Flüssigkeit setzt sich vorzugsweise aus Wasser, einwertigen und/oder mehrwertigen Alkolholen oder ihren Gemischen mit anderen hydrophilen Lösungsmitteln zusammen: Einwertige Alkohole sind vorzugsweise Methanol, Ethanol und Isopropanol. Mehrwertige Alkohole sind vorzugsweise Ethylenglykol, 1,2-Propandiol, 1,3-Propandiol, Glycerin, 1,3-Butandiol und Dipropylenglykol.
Um die erhaltene Emulsion zu stabilisieren, können weiterhin die dafür üblichen Mittel eingesetzt werden:
- Der äußeren oder der inneren Phase können Tenside beigemischt werden, die die Grenzflächenspannung herabsetzen. Dies können nichtionische, anionische, kationische und amphotere Tenside sein.
- Der inneren Phase können ionisch aktive Substanzen zugesetzt werden, die das Zusammenfließen der dispersen Phase durch elektrostatische Abstoßung behindern.
- Die Dichte der äußeren und der inneren Phase können durch geeignete Wahl der Lösungsmittel einander angeglichen werden, um ein Sedimentieren oder Aufrahmen der inneren Phase zu verzögern.

Daueben können der Phase A und/oder B Hilfsstoffe gemäß Anspruche 23 zugesetzt werden.

Zur Verbesserung der Scherwirkung bei der mechanischen Dispergierung kann die Viskosität der inneren Phase durch Verdickungsmittel in Form hydrophiler Polymere erhöht werden. Bei diesen Polymeren kann es sich um Vertreter folgender Gruppen handeln:
Polyvinylalkohole, teilhydrolysierte Polyvinylacetate, Polyethylenglykole, Polylactide, Polyvinylpyrrolidone, Poly(meth)acrylsäure und deren Salze sowie natürliche Pflanzenstärken und deren Derivate sowie Chitosane. Weiterhin kann es sich um Cellulosederivate handeln, insbesondere Methyl-, Carboxymethyl-, Hydroxymethyl-, Ethyl-, Hydroxyethyl- und Hydroxypropylcellulose.
Um einer unerwünschten Vermischung der inneren und äußeren Phase entgegenzuwirken, können in der inneren Phase dissoziierbare Salze gelöst werden, die die Polarität der inneren Phase heraufsetzen. Dies sind insbesondere die Halogenide der Alkali- und Erdalkalimetalle. Auch Harnstoff eignet sich wegen seines in der pharmazeutischen Wissenschaft bekannten Hydrotropieeffektes zu diesem Zweck.

Neben der fehlenden Mischbarkeit muß für beide Phasen erfindungsgemäß eine unterschiedliche Flüchtigkeit gewährleistet sein. Die Flüchtigkeit der äußeren Phase muß deutlich höher liegen als die der inneren Phase. Nur so wird ein im wesentlichen zweistufiger Trocknungsprozeß möglich. Die erste Stufe umfaßt die Entfernung des Lösungsmittels der äußeren Phase bis zu einer mechanischen Verfestigung dieser Phase. In der zweiten Stufe wird aus der bereits formstabil gewordenen äußeren Phase die innere durch Trocknen entfernt. Die Verfestigung der äußeren Phase kann durch Quervernetzungsprozesse unterstützt werden, die thermisch oder durch energiereiche Strahlung ausgelöst werden. Der Trocknungsvorgang erfolgt vorzugsweise zunächst auf einem niedrigen und anschließend auf einem höheren Temperaturniveau.
Die genügende Differenz der Flüchtigkeit ist praktisch immer gewährleistet, wenn die obengenannten Lösungsmittel für die äußere bzw. innere Phase verwendet werden. Eine Differenz der Siedebereiche beider Phasen kann hilfreich sein, sofern der Siedebereich der inneren Phase höher liegt. Den Siedebereichen bzw. Siedepunkten bei reinen Lösungsmitteln kommt jedoch eine geringere Bedeutung gegenüber der Flüchtigkeit zu, da technische Trocknungsprozesse hauptsächlich durch Verdunstung und weniger durch Sieden der flüchtigen Bestandteile erfolgen.

Die nach den vorstehenden Ausführungen erhaltene Emulsion wird schließlich auf eine geeignete Fläche oder einen kontinuierlich zugeführten bahnförmigen Träger in dünner Schicht, vorzugsweise 50 bis 2000 µm, aufgebracht. Dies geschieht durch ein geeignetes Auftraggerät, wie z.B. ein Walzenauftragwerk, einen Filmziehrahmen oder eine geeignete Extrusionsdüse.
Im Rahmen von Extrusionsprozessen wird die innere Phase vorzugsweise erst kurz vor der Extrusionsdüse in der äußeren Phase dispergiert.

Als Unterlage für das Beschichtungsverfahren von Polymerschäumen kommen vorzugsweise abweisend beschichtete bahnförmige Trägermaterialien, wie Folien aus Kunststoff oder Metall und dünnschichtige Papiere in Betracht. Von diesen Trägern kann die fertige Schaumschicht leicht abgelöst und der separaten Weiterverwendung zugeführt werden. Als Folienmaterial auf Kunststoffbasis können beispielsweise Polyethylenterephthalat, Polyethylen, Polypropylen, Polyvinylchlorid und Ethylvinylacetat eingesetzt werden. Die abweisende Beschichtung ist typischerweise auf Polyethylen, Polypropylen, Silikon oder einem fluorhaltigen Polymer aufgebaut.

Beim Aufbau mehrschichtiger Verbunde, z.B. transdermaler therapeutischer Systeme, wird die Schicht aus Polymerschaum typischerweise durch Umkaschieren weiterverarbeitet. Umkaschieren heißt, daß die Schaumschicht kontinuierlich flächig gegen ein anderes Flächenprodukt gepreßt wird. Die Schaumschicht bildet dabei einen dauerhaften mechanischen Verbund mit dem anderen Flächenprodukt. Dieser Verbund ist genügend fest, um anschließend den an dem Flächenprodukt anhaftenden Schaum von seinem Träger abziehen zu können.
Das bezeichnete Flächenprodukt besitzt typischerweise eine klebende oder haftklebende Oberfläche.

Die Trocknung erfolgt durch Umluft- oder Ablufttrocknung. Das Temperaturniveau der ersten Trocknungsphase liegt vorzugsweise zwischen 0 und 60°C. Die zweite Trocknungsphase erfolgt vorzugsweise bei 60-120°C. Der Temperaturverlauf kann auch mit einem gleitenden Übergang von niedrigen zu hohen Temperaturen gestaltet werden.

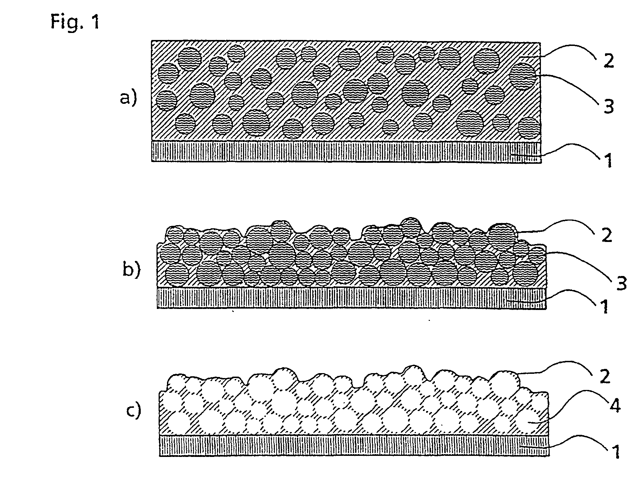

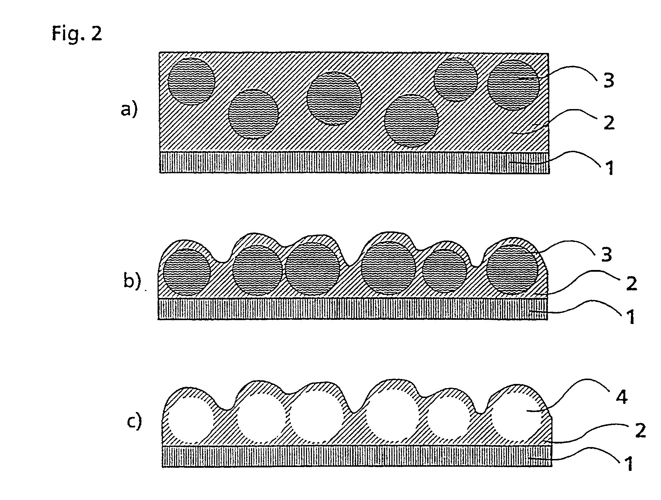

Eine besondere Anwendung des vorstehend beschriebenen Verfahrens stellt die Herstellung dünnschichtiger Schäume mit rauhen Oberflächen dar. Das bahnförmige Beschichtungsprodukt weist dabei nach der Trocknung an der freiliegenden Oberfläche eine Rauhigkeit auf, die durch Art und Menge der inneren Phase bei der Herstellung gesteuert werden kann. Zu diesem Zweck können zwei verschiedene Phänomene genutzt werden:
a) Der Volumenanteil der inneren Phase wird so gewählt, daß er während der ersten Phase des Trocknungsvorganges, also beim Einengen und Verfestigen der äußeren Phase, die maximale Packungsdichte überschreitet. Die als Ansammlung zusammengedrängten Tröpfchen der inneren Phase treten durch die bis dahin glatte Oberfläche reliefartig hervor. Dieser Vorgang ist vor, während und nach der Trocknung in Fig. 1a) bis c) dargestellt.
b) Die Tröpfchengröße der inneren Phase wird durch den Dispergierprozeß und gegebenenfalls grenzflächenaktive Zusatzstoffe so eingestellt, daß der mittlere Durchmesser der Tröpfchen größer, vorzugsweise um den Faktor 1,1 bis 1,5 höher, ist als die Schichtdicke des getrockneten Produktes.
   Bei entsprechender Führung des Trocknungsprozesses werden blasige Ausstülpungen an der Oberfläche des trockenen Produktes erhalten.
   Dieser Vorgang ist vor und nach der Trocknung in
   Fig. 2a) bis c) dargestellt.

In beiden Fällen wird ein Produkt erhalten, das sich wegen seiner rauhen Oberfläche besonders für die Weiterverarbeitung zu einem mehrschichtigen Verbund mit anderen bahnförmigen Polymermaterialien eignet:
Das nachträgliche Zusammenführen mehrerer bahnförmiger Polymerprodukte zu einem Mehrschichtprodukt geschieht typischerweise durch mechanisches Zusammenpressen der Schichten, z.B. durch ein Walzen-Kaschierwerk. Dieser Prozeß kann durch Erhitzen, gegebenenfalls bis zum Anschmelzen eines der Bestandteile unterstützt werden.

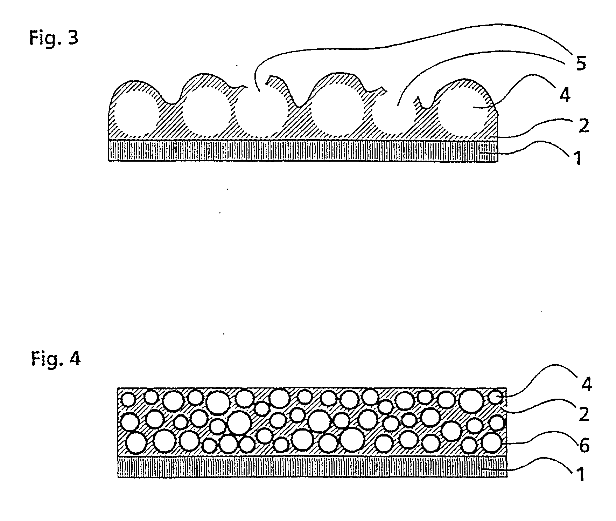

Bei der Herstellung transdermaler therapeutischer Systeme, medizinischer Pflaster oder medizinischer Bandagen besteht in der Regel zumindest eine der beteiligten Schichten aus einem Haftkleber, wodurch der Verbund mit anderen Schichten unterstützt wird. Dennoch erbringt der Prozeß des mechanischen Kaschierens häufig ungenügende Festigkeit oder Dauerhaftigkeit des Verbundes zwischen zwei Schichten, die unterschiedliche Polymere enthalten. Speziell bei TTS kann es nach der Anwendung beim Abziehen des Systems von der Haut zur Delaminierung kommen. Als unerwünschte Folge verbleibt die haftklebende Schicht ganz oder teilweise auf der Haut und nur die übrigen Schichten des TTS werden nach einem Bruch des Laminates abgezogen.
Der Verbund zwischen der Schicht eines Haftklebers und einer weiteren, nicht klebenden Polymerschicht kann erheblich verbessert werden, wenn diese Polymerschicht einen geschäumten Aufbau mit rauher Oberfläche besitzt. Bei der Kaschierung steht eine durch Rauhigkeit vergrößerte Grenzfläche zur Verfügung. Die rauhe Oberflächenstruktur ermöglicht außerdem eine Verzahnung beim mechanischen Zusammenpressen.
Diese Verzahnung wird insbesondere durch das Phänomen oberflächlich geplatzter Luftblasen des Schaumes gefördert. Die in Fig. 3 dargestellte Oberfläche des Schaumes weist offen zugängliche Hohlräume 5 auf, die die mechanische Verzahnung mit einer Schicht aus Fremdmaterial verbessern können.

Bei einer weiteren Anwendung des erfindungsgemäßen Verfahrens enthält die innere Phase neben flüchtigen Lösungsmitteln und gegebenenfalls den Dispersionsgrad beeinflussenden Mitteln zusätzlich noch schwerflüchtige Bestandteile.
Diese schwerflüchtigen Bestandteile verbleiben nach Abschluß des Trocknungsprozesses in den dispersen Hohlräumen des Schaumes (Fig.4). Typischerweise verbleiben diese Bestandteile in Form einer mehr oder weniger dicken inneren Auskleidung 6 der Wandung dieser Hohlräume. Die Polymerschaumschicht kann unter zusatz von hydrophilen Polymeren zur inneren Phase gebildete wandauskleidungen bzw Hohlkugeln, vorzugsweise in den in Anspruch 27 genannten Volumenanteilen, enthalten.
Die Auskleidungen können die mechanischen Eigenschaften des Schaumes verändern, z.B. zu einer Versteifung des Materials führen. Vorzugsweise enthalten diese Auskleidungen jedoch einen pharmazeutischen Wirkstoff in gelöster oder ungelöster Form oder sie bestehen sogar ganz aus einem solchen Wirkstoff. Der Wirkstoff kann dann über die sehr große innere Grenzfläche mit der äußeren Phase des Schaumes in Wechselwirkung treten, mit dem Ziel, durch die äußere Phase hindurch an daran angrenzende Verteilungsräume abgegeben zu werden.

### Anwendungsbeispiele:

Die 4 Beispiele weisen folgende Gemeinsamkeiten in der praktischen Durchführung auf:
Es wird eine Emulsion bereitet aus 2 verschiedenen Lösungen in einem bestimmten Gewichtsmengenverhältnis (GV) von A + B. Lösung A stellt die äußere und Lösung B die innere Phase dar. Die Emulsion wird mit einem Filmziehrahmen in definierter Schichtdicke (SD) auf eine geeignete bahnförmige Trägerfolie beschichtet. Der erhaltene Film wird nacheinander unter verschiedenen Trocknungsbedingungen (TB) getrocknet.

**Tabelle 1:**

| Nr. | Lösung A | Lösung B | GV | SD [µm] | Trägerfolie | TB 1 | TB 2 |
|---|---|---|---|---|---|---|---|
| 1 | Cariflex TR 16,0 1107 84,0 Benzin (80-110) | Propylenglykol | 7+2 | 400 | Hostaphan RN 100 silikonisiert | 10 Min. 60°C | 10 Min. 90°C |
| 2 | Cariflex TR 23,0 1107 Benzin 77,0 (80-110) | Mowiol 10-74 2,5 Wasser 7,5 Propylenglykol 90,0 | 6+1 | 500 | Hostaphan RN 100 silikonisiert | 10 Min. 60°C | 10 Min. 90°C |
| 3 | Cariflex TR 23,0 1107 77,0 Benzin (80-110) | Ethocel 70 1,0 Wasser 19,0 Ethanol 81,0 | 6+1 | 500 | Hostaphan RN 100 silikonisiert | 10 Min. Raumtemp. | 10 Min. 80°C |
| 4 | Silastic MDX 4 44,0 Polymer Silastic MDX 4 5,0 Härter Benzin 50,0 Tween 80 1,0 | Mowiol 8-88 5,0 Wasser 15,0 Propandiol 79,0 Methylenblau 1,0 | 4+1 | 250 | Scotchpak 1022 fluorpolymerbeschichtet | 10 Min. Raumtemp. | 10 Min. 100°C |

- Cariflex TR 1107: SIS-Blockcopolymer der Firma Shell
- Mowiol: Handelsname für verschiedene Polyvinylalkolhole der Firma Hoechst
- Silastic MDX 4: 2-Komponenten-Silikonkautschuk der Firma Dow Corning
- Ethocel: Handelsname für verschiedene Ethylcellulosen der Firma Dow Chemical Corp.
- Hostaphan: Handelsname für Polyethylenterephthalatfolien der Firma Hoechst
- Scotchpak: Handelsname für verschiedene Folien der Firma 3M
- Tween 80: nichtionisches Tensid
- Methylenblau: hydrophiler Farbstoff

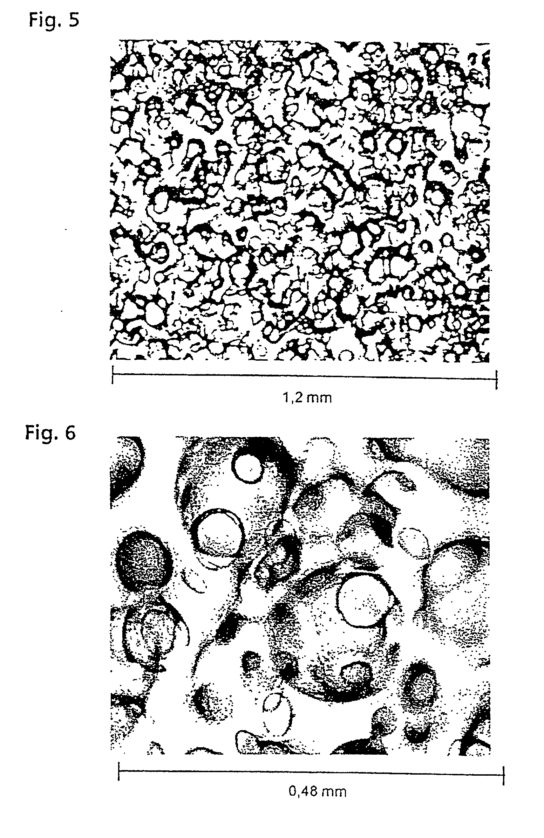

Das im Beispiel 1 erhaltene Produkt ist in Fig. 5 in 40facher mikroskopischer Vergrößerung dargestellt (ungefilterte Durchlichtaufnahme). Es handelt sich um eine dünne, schwammartige Schicht. Diese wird aus dem elastischen Polymer gebildet, in das in dichter Packung Hohlräume eingebettet sind. Die Oberfläche ist rauh und zerklüftet.

In der 100fachen Vergrößerung zeigen sich im Beispiel 2, bei sonst gleichartigem Erscheinungsbild wie Beispiel 1, kraterartige Öffnungen der Hohlräume, hin zur Oberfläche der getrockneten Schicht (Fig. 6). Dies entspricht dem in Fig. 3 theoretisch dargestellten Prinzip.

Beispiel 3 stellt eine Variante von Beispiel 2 dar, bei der mit milderen Trocknungsbedingungen gearbeitet werden kann.

In Beispiel 4 wird die Einbettung eines hydrophilen Polymers (Polyvinylalkohol) in Form von Hohlkugeln in die Matrix eines lipophilen, elastischen Silikonkautschuks gezeigt. Dies entspricht dem in Fig. 4 theoretisch dargestellten Phänomen.

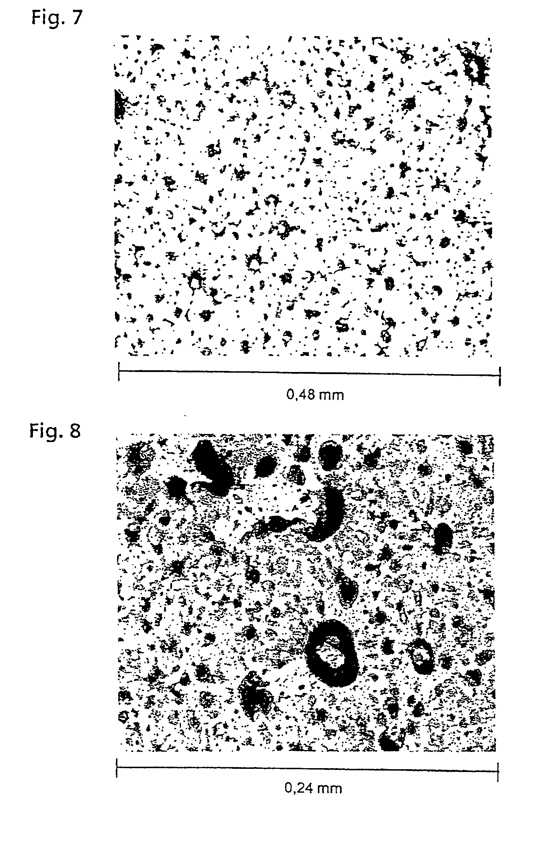

In Fig. 7 sind diese mit Methylenblau gefärbten Hohlkugeln in 100facher mikroskopischer Vergrößerung zu sehen. Die 200fache Vergrößerung (Fig. 8) zeigt anhand einiger größerer, zerborstener Objekte deutlich den Hohlkugelcharakter des dispers in Silikon eingebetteten und durch Methylenblau gefärbten Polyvinylalkohols. Methylenblau steht hier stellvertretend für einen pharmazeutischen Wirkstoff.

Anwendungsbeispiele zur Herstellung eines vollständigen TTS unter Einbindung eines Polymerschaums als Rückschicht: :

Transdermales therapeutisches System, aufgebaut aus einer dehäsiv ausgerüsteten Trägerschicht, mindestens einer wirkstoffhaltigen Schicht und einer praktisch wirkstoffundurchlässigen Rückschicht.

### Beispiel: TTS mit dem Wirkstoff Diclofenac Natriumsalz

Die haftklebende wirkstoffhaltige Schicht wird aus folgender Mischung hergestellt:

| | |
|---|---|
| Diclofenac Natriumsalz | 10,0 g |
| Laurinsäure | 8,3 g |
| Adipinsäure | 1,6 g |
| Eudragit E 100 | 14,3 g |
| Wasser | 64,9 g |
| Glycerin | 0,9 g |

Eudragit E 100 ist der Handelsname für ein basisches Ammoniomethacrylat-Copolymer der Firma Röhm Pharma.

Die flüssige Masse wird auf eine silikonisierte Polyethylenterephthalat-Folie (Hostaphan RN 100 µm der Firma Hoechst; Silikonisierung 54B durch Firma Laufenberg) in dünner Schicht ausgestrichen und 10 Minuten in einem Abluftofen bei 80°C getrocknet. Das Flächengewicht der getrockneten Haftkleberschicht beträgt 30 - 300 g/m², vorzugsweise 60 - 120 g/m².

Die offenliegende Haftkleberschicht dieses Vorproduktes wird mit einem gemäß Anwendungsbeispiel 2 treibmittelfrei hergestellten Polymerschaum mit rauher Oberfläche zusammenlaminiert. Der Laminierungsprozeß erfolgt durch mechanisches Zusammenpressen, vorzugsweise durch ein Walzenkaschierwerk.

Der Polymerschaum stellt die praktisch wirkstoffundurchlässige Rückschicht des TTS dar.

## Patentansprüche

1. Verfahren zur Herstellung von geschlossen-zelligen Polymerschaumschichten, dadurch gekennzeichnet, daß man eine ausbreit- bzw. ausstreichbare Emulsion aus einer Lösung eines oder mehrerer Polymere in einem oder mehreren organischen Lösungsmittel(n) als kohärente äußere Phase A und einer zu verflüchtigenden und mit der Phase A nicht mischbaren Flüssigkeit als disperse innere Phase B herstellt, wobei das Lösungsmittel oder die Lösungsmittelmischung der Phase A eine höhere Flüchtigkeit besitzt als die Flüssigkeit der Phase B, und anschließend die auf einer Unterlage schichtförmig ausgebreitete Emulsion einer Trocknung unterwirft, dadurch gekennzeichnet, daß in der ersten Stufe eine Verflüchtigung der Lösungsmittel aus der Phase A unter Ausbildung einer formbeständigen kohärenten äußeren Phase herbeigeführt wird, während in der zweiten Stufe eine Diffusion und anschließende Verdampfung bzw. Verdunstung der Flüssigkeit der inneren Phase unter zurücklassung von Hohlräumen erfolgt.

2. Verfahren nach Anspruch 1, dadurch gekennzeichnet, daß in der zweiten Stufe bei einer gegenüber der ersten Stufe erhöhten Temperatur getrocknet wird.

3. Verfahren nach Anspruch 1 od. 2, dadurch gekennzeichnet, daß in der ersten Stufe bei 0 - 60°C und gegebenenfalls in der zweiten Stufe bei 60 - 120°C getrocknet wird.

4. Verfahren nach einem oder mehreren der Ansprüche 1 bis 3, dadurch gekennzeichnet, daß eine Umluft- oder Ablufttrocknung durchgeführt wird.

5. Verfahren nach einem oder mehreren der vorangehenden Ansprüche, dadurch gekennzeichnet, daß als Unterlage ein gegebenenfalls abhäsiv ausgerüsteter bahnförmiger Träger verwendet wird.

6. Verfahren nach einem oder mehreren der vorangehenden Ansprüche, dadurch gekennzeichnet, daß die Emulsion auf der Unterlage in einer Schichtdicke von 50 - 2000 µm ausgebreitet wird.

7. Verfahren nach einem oder mehreren der vorangehenden Ansprüche, dadurch gekennzeichnet, daß bei der Verfestigung der Polymerphase A zusätzlich eine thermisch oder durch Strahlung ausgelöste Quervernetzung vorgesehen wird.

8. Verfahren nach einem oder mehreren der vorangehenden Ansprüche, dadurch gekennzeichnet, daß die Emulsion durch Extrusion der vorzugsweise kurz vor der Extrusionsdüse erzeugten Dispersion auggebreitet wird.

9. Verfahren nach einem oder mehreren der vorangehenden Ansprüche, dadurch gekennzeichnet, daß als Polymere vorzugsweise wasserunlösliche Elastomere verwendet werden.

10. Verfahren nach Anspruch 9, dadurch gekennzeichnet, daß als Elastomere Polyurethane, Ethylvinylacetat-Copolymere, Acrylatvinylpyrrolidon-Copolymere, Polyacrylate, Polymethacrylate, Polystyrol-basierte Kunstkautschuke, insbesondere SIS-Blockcopolymere und SBS-Blockcopolymere, reine Kohlenwasserstoffe wie Polyisobutylene, Polyisoprene und Silikonkautschuke verwendet werden.

11. Verfahren nach Anspruch 10, dadurch gekennzeichnet, daß haftklebende oder heißschmelzklebende Modifizierungen oder Mischungen der Elastomeren verwendet werden.

12. Verfahren nach einem oder mehreren der vorangehenden Ansprüche, dadurch gekennzeichnet, daß als organische Lösungsmittel der Phase A Isopropanol, Aceton, Methylethylketon, Ethylacetat, Diethylether, Diisopropylether, Cyclohexan, Hexan, Heptan, Octan, Petrolether, Toluol und Benzin verschiedener Siedebereiche verwendet werden.

13. Verfahren nach einem oder mehreren der vorangehenden Ansprüche, dadurch gekennzeichnet, daß für die Bildung der Phase B hydrophile Lösungsmittel, insbesondere Wasser, einwertige und/oder mehrwertige Alkohole verwendet werden.

14. Verfahren nach einem oder mehreren der vorangehenden Ansprüche, dadurch gekennzeichnet, daß der inneren Phase B als Verdickungsmittel hydrophile Polymere zur Verbesserung der Dispergierbarkeit zugesetzt werden, die zumindest einen Vertreter aus der Gruppe der Polyvinylalkohole, teilhydrolysierten Polyvinylacetate, Polyethylenglykole, Polylactide, Polyvinylpyrrolidone, Poly(meth)acrylsäure und deren Salzen, natürlichen Pflanzenstärken und deren Derivaten, Chitosanen sowie Cellulosederivaten, insbesondere Methyl-Carboxymethyl-, Hydroxymethyl-, Ethyl-, Hydroxethyl- und Hydroxypropylcellulose enthalten.

15. Verfahren nach einem, oder mehreren der vorangehenden Ansprüche, dadurch gekennzeichnet, daß den Phasen A und/oder B Tenside zugesetzt werden.

16. Verfahren nach einem oder mehreren der vorangehenden Ansprüche, dadurch gekennzeichnet, daß der inneren Phase B ionisch aktive Substanzen zugesetzt werden.

17. Verfahren nach einem oder mehreren der vorangehenden Ansprüche, dadurch gekennzeichnet, daß eine Aufrauhung der Produktoberfläche durch Auswahl eines entsprechend hohen Volumenanteils der Phase B oder dadurch bewirkt wird, daß der mittlere Durchmesser der Phase B-Tröpfchen größer ist als die Schichtdicke des getrockneten Produkts.

18. Verfahren nach Anspruch 17, dadurch gekennzeichnet, daß der Durchmesser um den Faktor 1,1 - 1,5 höher ist als die Schichtdicke.

19. Verfahren nach einem oder mehreren der vorangehenden Ansprüche, dadurch gekennzeichnet, daß eine Aufrauhung der Produktoberfläche durch Aufsprengen von Oberflächen-Hohlräumen bewirkt wird.

20. Verfahren zur Herstellung von geschlossen-zelligen Polymerschaumschichten mit einer ausbreit- bzw. ausstreichbaren Emulsion aus einer Lösung eines oder mehrerer Polymere in einem oder mehreren organischen Lösungsmittel (n) als kohärente äußere Phase A und mit einer zu verflüchtigenden und mit der Phase A nicht mischbaren Flüssigkeit als disperse innere Phase B, wobei das Lösungsmittel oder die Lösungsmittelmischung der Phase A eine höhere Flüchtigkeit besitzt als die Flüssigkeit der Phase B bei dem die Emulsion auf einer Unterlage schichtförmig ausgebreitet und einer Trocknung unterworfen wird, bei der in der ersten Stufe eine Verflüchtigung der Lösungsmittel aus der Phase A unter Ausbildung einer formbeständigen kohärenten äußeren Phase herbeigeführt wird, dadurch gekennzeichnet, daß der Phase B schwerflüchtige Bestandteile zugemischt werden, die nach einer zweiten Trocknungsstufe eine Wandauskleidung der verbleibenden Hohlräume bilden.

21. Verfahren nach Anspruch 20, dadurch gekennzeichnet, daß als schwerflüchtige Bestandteile hydrophile Polymere zugemischt werden.

22. Verfahren nach Anspruch 20 oder 21, dadurch gekennzeichnet, daß die schwerflüchtigen Bestandteile pharmazeutische Wirkstoffe sind oder solche enthalten.

23. Verfahren nach einem oder mehreren der vorangehenden Ansprüche, dadurch gekennzeichnet, daß Phase A und/oder B pharmazeutische Wirkstoffe "und/oder Hilfsstoffe aus der Gruppe der Tackifier, Enhancer, Weichmacher, Kohäsionsverbesserer, Sorbentien, Antioxidantien, Metallionenkomplexierer, Lichtschutzstoffe, Farbstoffe, Pigmente und/oder Aufheller zugesetzt werden.

24. Dermal applizierbare therapeutische Systeme, insbesondere Dermalpflaster, zur Verabreichung von Wirkstoffen an bzw. über die Haut, gekennzeichnet durch zumindest eine nach einem der vorangehenden Ansprüche erhaltene Polymerschaumschicht.

25. Therapeutische Systeme nach Anspruch 24, dadurch gekennzeichnte , daß die kohärente Phase durch einen Haftkleber gebildet wird und bei Applikation der Haut zugewandt ist.

26. Therapeutische Systeme nach Anspruch 24 oder 25, dadurch gekennzeichnet, daß die Polymerschaumschicht unter Zusatz von hydrophilen Polymeren zur Phase B gebildete Wandauskleidungen bzw. Hohlkugeln enthält.

27. Therapeutische Systeme nach Anspruch 26, dadurch gekennzeichnet, daß der Volumenanteil der Hohlkugeln in der Polymerschaumschicht bei 5 - 95 %, vorzugsweise 10 - 50 %, und der Anteil des wandbildenden Polymeren am Hohlkugelvolumen bei 1 - 90 %, vorzugsweise 5 - 50 %, liegt.

## Claims

1. Process for producing closed-cell polymer foam layers, characterized in that a spreadable emulsion is prepared from a solution of one or more polymers in one or more organic solvents as coherent outer phase A and from a liquid intended for volatilization and immiscible with phase A, as disperse inner phase B, the solvent or solvent mixture of phase A possessing a higher volatility than the liquid of phase B, and that subsequently the emulsion, spread out in layer form on a substrate, is subjected to drying, characterized in that in the first stage, volatilization of the solvents from phase A is induced, with the formation of a dimensionally stable, coherent outer phase, while in the second stage diffusion and subsequent evaporation of the liquid of the inner phase takes place, leaving voids behind.

2. Process according to Claim 1, characterized in that drying in the second stage takes place at a temperature higher than that of the first stage.

3. Process according to Claim 1 or 2, characterized in that drying takes place in the first stage at 0 - 60°C and, if appropriate, in the second stage at 60 - 120°C.

4. Process according to one or more of Claims 1 to 3, characterized in that drying is conducted with air circulation or air removal.

5. Process according to one or more of the preceding claims, characterized in that a weblike support with or without abhesive treatment is used as substrate.

6. Process according to one or more of the preceding claims, characterized in that the emulsion is spread on the substrate in a layer thickness of 50 - 2000 µm.

7. Process according to one or more of the preceding claims, characterized in that crosslinking initiated thermally or by radiation is additionally provided during the setting of the polymer phase A.

8. Process according to one or more of the preceding claims, characterized in that the emulsion is spread by extruding the dispersion, which is preferably produced shortly before the extrusion die.

9. Process according to one or more of the preceding claims, characterized. in that preferably water-insoluble elastomers are used as polymers.

10. Process according to Claim 9, characterized in that polyurethanes, ethylene-vinyl acetate copolymers, acrylatevinylpyrrolidone copolymers, polyacrylates, polymethacrylates, polystyrene-based rubbers, especially SIS block copolymers and SBS block copolymers, pure hydrocarbons such as polyisobutylenes, polyisoprenes, and also silicone rubbers are used as elastomers.

11. Process according to Claim 10, characterized in that elastomer forms modified for pressure-sensitive adhesion or hot-melt adhesion, or mixtures of the elastomers, are used.

12. Process according to one or more of the preceding claims, characterized in that isopropanol, acetone, methyl ethyl ketone, ethyl acetate, diethyl ether, diisopropyl ether, cyclohexane, hexane, heptane, octane, petroleum ether, toluene and mineral spirits of different boiling ranges are used as organic solvents of phase A.

13. Process according to one or more of the preceding claims, characterized in that hydrophilic solvents, especially water, monohydric and/or polyhydric alcohols, are used for the formation of phase B.

14. Process according to one or more of the preceding claims, characterized in that hydrophilic polymers are added as thickeners to the inner phase B in order to improve the dispersibility, the said polymers comprising at least one representative from the group consisting of polyvinyl alcohols, partially hydrolysed polyvinyl acetates, polyethylene glycols, polylactides, polyvinylpyrrolidones, poly(meth)acrylic acid and salts thereof, natural vegetable starches and derivatives thereof, chitosans and cellulose derivatives, especially methyl-, carboxymethyl-, hydroxymethyl-, ethyl-, hydroxyethyl- and hydroxypropylcellulose.

15. Process according to one or more of the preceding claims, characterized in that surfactants are added to phase A and/or phase B.

16. Process according to one or more of the preceding claims, characterized in that substances having ionic activity are added to the inner phase B.

17. Process according to one or more of the preceding claims, characterized in that the product surface is roughened by selecting an appropriately high volume fraction of phase B or by ensuring that the average diameter of the phase B droplets is greater than the layer thickness of the dried product.

18. Process according to Claim 17, characterized in that the diameter is greater by a factor of 1.1 - 1.5 than the layer thickness.

19. Process according to one or more of the preceding claims, characterized in that the product surface is roughened by the exploding of surface voids.

20. Process for producing closed-cell polymer foam layers comprising a spreadable emulsion prepared from a solution of one or more polymers in one or more organic solvents as coherent outer phase A and comprising a liquid intended for volatilization and immiscible with phase A, as disperse inner phase B, the solvent or solvent mixture of phase A possessing a higher volatility than the liquid of phase B, in which process the emulsion is spread out in layer form on a substrate and subjected to drying during which, in the first stage, volatilization of the solvents from phase A is induced, with the formation of a dimensionally stable, coherent outer phase, characterized in that constituents of low volatility which following the second drying stage form a lining on the walls of the remaining voids are admixed to phase B.

21. Process according to Claim 20, characterized in that hydrophilic polymers are admixed as low-volatility constituents.

22. Process according to Claim 20 or 21, characterized in that the low-volatility constituents consist of or comprise pharmaceutical active substances.

23. Process according to one or more of the preceding claims, characterized in that pharmaceutical active substances and/or auxiliaries selected from the group consisting of tackifiers, enhancers, plasticizers, cohesion enhancers, sorbents, antioxidants, metal ion complexing agents, photoprotectants, dyes, pigments and brighteners are added to phase A and/or phase B.

24. Dermally applicable therapeutic systems, especially dermal patches, for administering active substances to and/or through the skin, characterized by at least one polymer foam layer obtained in accordance with one of the preceding claims.

25. Therapeutic systems according to Claim 24, characterized in that the coherent phase is formed by a pressure-sensitive adhesive and faces the skin on application.

26. Therapeutic systems according to Claim 24 or 25, characterized in that the polymer foam layer includes hollow beads and/or wall linings formed by addition of hydrophilic polymers to phase B.

27. Therapeutic systems according to Claim 26, characterized in that the volume fraction of the hollow beads in the polymer foam layer is 5 - 95%, preferably 10 - 50%, and the fraction of the wall-forming polymer in the total hollow-bead volume is 1 - 90%, preferably 5 - 50%.

## Revendications

1. Procédé pour la préparation de couches de mousse polymère à alvéoles fermés, caractérisé en ce qu'on prépare une émulsion que l'on peut étendre, respectivement que l'on peut étaler, à partir d'une solution d'un ou de plusieurs polymères dans un ou plusieurs solvants organiques, à titre de phase externe cohérente A et à partir d'un liquide à volatiliser et non miscible avec la phase A, à titre de phase interne dispersée B, dans lequel le solvant ou le mélange de solvants de la phase A possède une volatilité supérieure à celle du liquide de la phase B, et on soumet ensuite à un séchage l'émulsion étendue sous la forme d'une couche sur un substrat, caractérisé en ce qu'on obtient, dans la première étape, une volatilisation des solvants de la phase A avec formation d'une phase externe cohérente résistant à la déformation, tandis que dans la seconde étape a lieu une diffusion et une évaporation respectivement une vaporisation ultérieure du liquide de la phase interne en laissant subsister des vacuoles.

2. Procédé selon la revendication 1, caractérisé en ce que, dans la seconde étape, on sèche à une température supérieure à celle de la première étape.

3. Procédé selon la revendication 1 ou 2, caractérisé en ce que, dans la première étape, on sèche à une température de 0 à 60°C et le cas échéant, dans la seconde étape, on sèche à une température de 60 à 120°C.

4. Procédé selon une ou plusieurs des revendications 1 à 3, caractérisé en ce qu'on procède à un séchage avec circulation d'air ou avec évacuation d'air.

5. Procédé selon une ou plusieurs des revendications précédentes, caractérisé en ce qu'on utilise à titre de substrat, un support en forme de bande, le cas échéant rendu anti-adhésif.

6. Procédé selon une ou plusieurs des revendications précédentes,
caractérisé en ce qu'on étend l'émulsion sur le substrat en une épaisseur de couche de 50 à 2000 µm.

7. Procédé selon une ou plusieurs des revendications précédentes, caractérisé en ce qu'on prévoit, lors de la consolidation de la phase polymère A, en outre une réticulation déclenchée par voie thermique ou par exposition à un rayonnement.

8. Procédé selon une ou plusieurs des revendications précédentes, caractérisé en ce qu'on étend l'émulsion par extrusion de la dispersion formée de préférence à un endroit situé peu avant la filière d'extrusion.

9. Procédé selon une ou plusieurs des revendications précédentes, caractérisé en ce qu'on utilise, à titre de polymères, de préférence, des élastomères insolubles dans l'eau.

10. Procédé selon la revendication 9, caractérisé en ce qu'on utilise, à titre d'élastomères, des polyuréthanes, des copolymères d'éthylène-acétate de vinyle, des copolymères d'acrylate-vinylpyrrolidone, des polyacrylates, des polyméthacrylates, des caoutchoucs à base de polystyrène, en particulier des copolymères séquencés de type SIS et des copolymères séquencés de type SBS, des hydrocarbures purs tels que des polyisobutylènes, des polyisoprènes et des caoutchoucs de silicone.

11. Procédé selon la revendication 10, caractérisé en ce qu'on utilise des modifications adhésives ou thermofusibles des élastomères ou encore des mélanges de ces derniers.

12. Procédé selon une ou plusieurs des revendications précédentes, caractérisé en ce qu'on utilise, à titre de solvants organiques de la phase A, de l'isopropanol, de l'acétone, de la méthyléthylcétone, de l'acétate d'éthyle, de l'éther diéthylique, de l'éther diisopropylique, du cyclohexane, de l'hexane, de l'heptane, de l'octane, de l'éther de pétrole, du toluène et de l'essence de différents domaines d'ébullition.

13. Procédé selon une ou plusieurs des revendications précédentes, caractérisé en ce qu'on utilise, pour la formation de la phase B, des solvants hydrophiles, en particulier de l'eau, des alcools monovalents et/ou polyvalents.

14. Procédé selon une ou plusieurs des revendications précédentes, caractérisé en ce qu'on ajoute, à la phase interne B, à titre d'épaississant, des polymères hydrophiles pour améliorer l'aptitude à la mise en dispersion, qui contiennent au moins un représentant choisi parmi le groupe comprenant des alcools polyvinyliques, des acétates de polyvinyle partiellement hydrolysés, des polyéthylèneglycols, des polylactides, de la polyvinylpyrrolidone, de l'acide poly(méth)acrylique et ses sels, des amidons végétaux naturels et leurs dérivés, des chitosanes, ainsi que des dérivés de la cellulose, en particulier la méthyl-carboxyméthylcellulose, l'hydroxyméthylcellulose, l'éthylcellulose, l'hydroxy-éthylcellulose et l'hydroxypropylcellulose.

15. Procédé selon une ou plusieurs des revendications précédentes, caractérisé en ce qu'on ajoute des agents tensioactifs à la phase A et/ou à la phase B.

16. Procédé selon une ou plusieurs des revendications précédentes, caractérisé en ce qu'on ajoute, à la phase interne B, des substances à activité ionique.

17. Procédé selon une ou plusieurs des revendications précédentes, caractérisé en ce qu'on met en oeuvre un procédé consistant à rendre rugueuse la surface du produit en sélectionnant une fraction volumique élevée correspondante de la phase B ou en rendant le diamètre moyen des gouttelettes de la phase B supérieur à l'épaisseur de couche du produit séché.

18. Procédé selon la revendication 17, caractérisé en ce que le diamètre est supérieur du facteur de 1,1 à 1,5 à l'épaisseur de couche.

19. Procédé selon une ou plusieurs des revendications précédentes, caractérisé en ce qu'on met en oeuvre le procédé consistant à rendre rugueuse la surface du produit en faisant sauter des vacuoles de la surface.

20. Procédé pour la préparation de couches de mousse polymère à alvéoles fermés avec une émulsion que l'on peut étendre, respectivement que l'on peut étaler, à partir d'une solution d'un ou de plusieurs polymères dans un ou plusieurs solvants organiques, à titre de phase externe cohérente A et avec un liquide à volatiliser et non miscible avec la phase A, à titre de phase interne dispersée B, dans lequel le solvant ou le mélange de solvants de la phase A possède une volatilité supérieure à celle du liquide de la phase B, dans lequel on étend l'émulsion sous la forme d'une couche sur un substrat et on la soumet ensuite à un séchage au cours duquel on obtient, dans la première étape, une volatilisation des solvants de la phase A avec formation d'une phase externe cohérente résistant à la déformation, caractérisé en ce qu'on ajoute par mélange à la phase B des constituants de faible volatilité qui forment, après une seconde étape de séchage, un garnissage des parois des vacuoles qui subsistent.

21. Procédé selon la revendication 20, caractérisé en ce qu'on ajoute par mélange à titre de constituants de faible volatilité, des polymères hydrophiles.

22. Procédé selon la revendication 20 ou 21, caractérisé en ce que les constituants de faible volatilité sont des principes actifs pharmaceutiques ou contiennent ces derniers.

23. Procédé selon une ou plusieurs des revendications précédentes, caractérisé en ce qu'on ajoute à la phase A et/ou à la phase B, des principes actifs pharmaceutiques et/ou des adjuvants choisis parmi le groupe comprenant des agents rendant adhésif, des agents d'activation, des plastifiants, des agents améliorant la cohésion, des adsorbants, des antioxydants, des agents complexant les ions métalliques, des substances de protection contre l'effet de la lumière, des colorants, des pigments et/ou des agents de blanchiment optique.

24. Systèmes thérapeutiques aptes à être appliqués sur la peau, en particulier emplâtres dermiques pour l'administration de principes actifs sur, respectivement via la peau, caractérisés par au moins une couche de mousse polymère obtenue conformément à l'une quelconque des revendications précédentes.

25. Systèmes thérapeutiques selon la revendication 24, caractérisés en ce qu'on forme la phase cohérente à l'aide d'un adhésif et on la toume vers la peau lors de l'application.

26. Systèmes thérapeutiques selon la revendication 24 ou 25, caractérisés en ce que la couche de mousse polymère contient des billes creuses, respectivement des garnissages de parois obtenus par addition de polymères hydrophiles à la phase B.

27. Systèmes thérapeutiques selon la revendication 26, caractérisés en ce que la fraction volumique des billes creuses dans la couche de mousse polymère s'élève de 5 à 95 % de préférence de 10 à 50 % et la fraction du polymère formateur de paroi rapportée au volume des billes creuses s'élève de 1 à 90 %, de préférence de 5 à 50 %.
